# EUROPÄISCHE PATENTSCHRIFT

(11) **EP 2 542 781 B1**
(45) Veröffentlichungstag und Bekanntmachung des Hinweises auf die Patenterteilung: **19.03.2014**
(21) Anmeldenummer: 11703212.8
(22) Anmeldetag: 08.02.2011
(51) Int. Cl.: F04B 43/12, A61M 5/142

(54) **SCHLAUCHPUMPE**
PERISTALTIC PUMP
POMPE A TUYAU

(30) Priorität: 01.03.2010 DE 102010000594
(43) Veröffentlichungstag der Anmeldung: 09.01.2013
(73) Patentinhaber: Ulrich GmbH & Co. KG, 89081 Ulm (DE)
(72) Erfinder: RODAU, Sergej, 06849 Dessau (DE); BAECKE, Martin, 06947 Dessau (DE); SCHWERDTFEGER, Uwe, 39439 Amesdorf (DE)
(74) Vertreter: Charrier, Rapp & Liebau
(86) Internationale Anmeldenummer: PCT/EP2011/051815
(87) Internationale Veröffentlichungsnummer: WO 2011/107326

(56) Entgegenhaltungen:
- GB-A- 915 959
- US-A- 4 174 193

## Beschreibung

Die Erfindung betrifft eine Schlauchpumpe nach dem Oberbegriff des Anspruchs 1.

Derartige Schlauchpumpen werden insbesondere im Bereich der Medizintechnik, beispielsweise als Infusionspumpe oder in Injektions- und Dialysegeräten verwendet. Gattungsgemäße Schlauchpumpen zur Verwendung in Kontrastmittel-Injektionsgeräten sind in der EP 2 011 541 A2 beschrieben, darunter ein vorbenutztes Injektionsgerät der Anmelderin, welches unter der Kennzeichnung "ulrich missouri/mississippi" im Markt erhältlich ist. Dieses vorbekannte Kontrastmittel-Injektionsgerät umfasst eine gattungsgemäße Schlauchpumpe mit drei in Form von Quetschrollen ausgebildeten Quetschelementen, welche einen in die Schlauchpumpe eingeführten Schlauch, in dem das Kontrastmittel oder eine NaCl-Spüllösung geführt wird, unter Quetschung des Schlauchs gegen ein verschwenkbares Gegenlager drückt und dadurch das in dem Schlauch geführte Medium in Förderrichtung weiter fördert. Die Quetschrollen sind dabei frei drehbar auf einer rotierend angetriebenen Trägerscheibe angeordnet. Bei laufender Pumpe dreht sich die Trägerscheibe im Uhrzeigersinn und die auf der Trägerscheibe angeordneten Quetschrollen drehen sich entgegen dem Uhrzeigersinn. Die Drehbewegung der Trägerscheibe und der Quetschrollen wird von einem Antriebsmotor und einem Getriebe, an welches die Trägerscheibe und die Quetschrollen gekoppelt sind, vermittelt. Das Gegenlager ist zweiteilig ausgebildet und besteht aus zwei kreissegmentförmigen Gegenlager-Segmenten, welche jeweils an einer schwenkbaren Klappe angeordnet sind. Die beiden schwenkbaren Klappen sind um eine gemeinsame Schwenkachse gegeneinander verschwenkbar. Zum Einführen des Schlauchs in die Schlauchpumpe werden die beiden Klappen zunächst auseinander geschwenkt, so dass der Schlauch zwischen dem Außenumfang der Quetschrollen und den mit den Klappen von den Quetschrollen weggeschwenkten Gegenlager-Segmenten manuell eingeführt werden kann. Vor dem Einfädeln des Schlauchs wird die Pumpe manuell in eine für das Einfädeln des Schlauchs geeignete Stellung gedreht, so dass der Schlauch zwischen dem Außenumfang der Quetschrollen und den ausgeschwenkten Gegenlager-Segmenten eingefädelt werden kann. Nach dem Einfädeln des Schlauchs werden die beiden Klappen wieder zusammengeschwenkt, wodurch die beiden Gegenlager-Segmente in Anlage an den Schlauch gebracht werden und diesen gegen den Außenumfang der Quetschrollen drücken. Nach dem Einschwenken der beiden verschwenkbaren Klappen mit den daran angeordneten Gegenlager-Segmenten werden die beiden Klappen verriegelt und die Schlauchpumpe kann zur Förderung des in dem Schlauch geführten Mediums in Drehung versetzt werden.

Aus der US 4 174 193 A ist eine Schlauchpumpe zur Förderung eines in einem Schlauch geführten Mediums bekannt, welche mehrere Quetschrollen umfasst, welche den Schlauch gegen ein Gegenlager drücken.

Die Einführung des Schlauchs in die Schlauchpumpe erweist sich allerdings als umständlich und zeitaufwendig. Insbesondere das Wegschwenken und Zusammenschwenken der beiden verschwenkbaren Klappen und das Drehen der Pumpe in eine zum Einfädeln des Schlauchs geeignete Stellung erweist sich als zeitaufwendig. Das Einfädeln des Schlauchs erfordert vom Bediener darüber hinaus eine gewisse Geschicklichkeit.

Hiervon ausgehend liegt der Erfindung die Aufgabe zugrunde, eine gattungsgemäße Schlauchpumpe so weiterzubilden, dass eine einfachere und schnellere Einführung eines Schlauchs in die Schlauchpumpe ermöglicht wird.

Diese Aufgabe wird mit einer Schlauchpumpe mit den Merkmalen des Anspruchs 1 gelöst. Bevorzugte Ausführungsformen dieser Schlauchpumpe sind den Unteransprüchen zu entnehmen.

Die erfindungsgemäße Schlauchpumpe verfügt über eine Einfädeleinrichtung zum Einfädeln des Schlauchs zwischen den Quetschelementen und dem Gegenlager. Mit der Einfädeleinrichtung wird der Schlauch automatisch in die Schlauchpumpe eingeführt. Es ist lediglich noch erforderlich, dass der Bediener den schlaufenförmig gebogenen Schlauch in die Einfädeleinrichtung einlegt und die Schlauchpumpe startet. Die Einfädeleinrichtung wird mit der Schlauchpumpe in Gang gesetzt und führt den Schlauch automatisch in die Schlauchpumpe zwischen den Quetschelementen und dem Gegenlager ein, wobei durch die Pumpenbewegung der Schlauch zwischen den Quetschelementen und dem Gegenlager eingequetscht und in Förderrichtung der Schlauchpumpe weiter eingezogen wird, bis der Schlauch vollständig in die Schlauchpumpe eingezogen ist.

Bevorzugt umfasst die Einfädeleinrichtung eine auf einer Welle angeordnete Schneckenspindel. Die Welle ist dabei drehbar in einem Gehäuseteil der Schlauchpumpe gelagert und wird von einem Spindelantrieb in Drehung versetzt. Der Spindelantrieb ist zweckmäßig mit dem Antriebsmotor der Schlauchpumpe gekoppelt, so dass der Spindelantrieb zusammen mit dem Antriebsmotor in Gang gesetzt wird, sobald die Schlauchpumpe eingeschaltet wird. Beim Einschalten der Schlauchpumpe führt die Einfädeleinrichtung einen in den obersten Schneckengang der Schneckenspindel eingelegten Schlauch zunächst nach unten in Richtung der Ebene, in der sich die Quetschrollen und das Gegenlager befinden und fädelt den Schlauch dann automatisch in die Schlauchpumpe zwischen den Quetschelementen und dem Gegenlager ein. Durch die vom Antriebsmotor vermittelte Bewegung der Schlauchpumpe wird der Schlauch schließlich vollständig in die Schlauchpumpe entlang der gesamten Länge bzw. des gesamten Umfangs des Gegenlagers eingezogen.

Um den Schlauch in die Einfädeleinrichtung einlegen zu können, ist zweckmäßig eine Fixiereinrichtung zur Fixierung des Schlauchs vorgesehen, wobei die Fixiereinrichtung bevorzugt eine Fixierung des Schlauchs an einer ersten, eingangsseitig der Schlauchpumpe angeordneten Stelle und wenigstens einer zweiten, ausgangsseitig angeordneten Stelle der Schlauchpumpe ermöglicht. Nach dem Einlegen des Schlauchs in die Fixiereinrichtung verläuft der an den beiden Fixierstellen fixierte Schlauch zwischen den beiden Fixierstellen schlaufenförmig.

In einem bevorzugten Ausführungsbeispiel ist die Schlauchpumpe als Rotations-Schlauchpumpe ausgebildet, wobei die Quetschelemente durch Quetschrollen gebildet sind, welche drehbar auf einer Trägerscheibe gelagert sind. Die Trägerscheibe und die Quetschrollen werden von einem Antriebsmotor bei laufender Pumpe in Drehung versetzt. Die Bewegung des Antriebsmotors wird dabei zweckmäßig über ein Planetengetriebe auf die Trägerscheibe und die Quetschrollen übertragen, so dass auch bei nicht eingelegtem Schlauch sowohl die Trägerscheibe als auch die darin drehbar gelagerten Quetschrollen in Rotation versetzt werden können. Beim Starten der Schlauchpumpe wird somit auch bei noch nicht eingeführtem Schlauch die Trägerscheibe in Drehung versetzt. Gleichzeitig fädelt die Einfädeleinrichtung den darin eingefügten Schlauch in die Schlauchpumpe ein, indem sie den Schlauch in Richtung eines der Einfädeleinrichtung benachbarten Quetschelements (Quetschrolle) und dem diesen gegenüberliegenden Gegenlager führt.

Um die selbsttätige Einfädelung des Schlauchs in die Schlauchpumpe durch die Einfädeleinrichtung zu unterstützten ist neben den Quetschrollen zusätzlich noch wenigstens eine Führungsrolle vorgesehen. Bevorzugt sind mehrere Führungsrollen vorgesehen, welche jeweils zwischen benachbarten Quetschrollen auf der Trägerscheibe drehbar angeordnet sind.

Die Führungsrollen weisen am Außenumfang eine umlaufende Nut 34 auf, in welche der Schlauch eingreifen kann. Wenn der Schlauch von der Einfädeleinrichtung nach unten in Richtung der Trägerscheibe geführt wird, greift er in die Nut am Außenumfang derjenigen Führungsrolle ein, die gerade benachbart zur Einfädeleinrichtung liegt. Durch die Drehung der Trägerscheibe bewegt sich die darauf angeordnete Führungsrolle in Förderrichtung der Pumpe weiter und zieht dabei den Schlauch zum einen nach unten in Richtung der Trägerscheibe und drückt ihn durch Formschluss zum anderen in radialer Richtung nach außen gegen das Gegenlager. Bei weiterer Drehung der Trägerscheibe zieht die Führungsrolle den Schlauch aufgrund der Haftreibung an der Schlauchoberfläche und des Kraftschlusses in der Nut an ihrem Außenumfang weiter in die Schlauchpumpe entlang des Innenumfangs des kreissegmentförmig ausgebildeten Gegenlagers ein, bis die Trägerscheibe mit der darauf angeordneten Führungsrolle (nahezu) eine volle Umdrehung ausgeführt hat und der Schlauch durch die weitere Drehung der Trägerscheibe vollständig in die Schlauchpumpe eingezogen ist.. Durch die Drehung der Trägerscheibe wird der Schlauch schließlich von der auf der Trägerscheibe der Führungsrolle folgenden Quetschrolle gegen das Gegenlager gequetscht und so zwischen der Quetschrolle und dem Gegenlager geklemmt.

Um zu gewährleisten, dass auch bei noch nicht eingeführtem Schlauch sowohl die Trägerscheibe als auch die darin drehbar gelagerten Quetschrollen in Rotation versetzt werden können, ist ein Getriebe mit einem drehfest mit einer Antriebswelle des Antriebs verbunden Sonnenrad und einem drehfest mit der jeweiligen Quetschrolle verbundenen ersten Planetenrad sowie ein Planetengetriebe vorgesehen, welches das Sonnenrad und wenigstens ein Planetenrad für jede Quetschrolle umfasst, welches mit dem als Hohlrad wirkenden Innenumfang des Pumpengehäuses gekoppelt ist. Dadurch wird das von der Antriebswelle auf das Sonnenrad übertragene Drehmoment vom Sonnenrad über den als Hohlrad wirkenden Innenumfang des fest stehenden Pumpengehäuses auf die Trägerscheibe übertragen, wodurch die Trägerscheibe bei laufender Pumpe auch ohne einen eingelegten Schlauch in Drehung versetzt wird.

Weitere Vorteile und Besonderheiten der Erfindung ergeben sich aus dem nachfolgend unter Bezugnahme auf die begleitenden Zeichnungen beschriebenen Ausführungsbeispiel. Die Zeichnungen zeigen:
- **Figur 1:**: Draufsicht auf eine Injektionsvorrichtung, in der eine erfindungsgemäße Schlauchpumpe verwendet wird;
- **Figur 2:**: Perspektivische Darstellung einer erfindungsgemäßen Schlauchpumpe;
- **Figur 3:**: Explosionsdarstellung der Schlauchpumpe von Figur 2;
- **Figur 4a:**: Schnittdarstellung der Schlauchpumpe von Figur 2 entlang der Ebene A-A;
- **Figur 4b:**: Detailansicht einer Schnittdarstellung der Schlauchpumpe von Figur 2 im Bereich des Gegenlagers und einer dem Gegenlager gegenüberliegenden Führungsrolle;

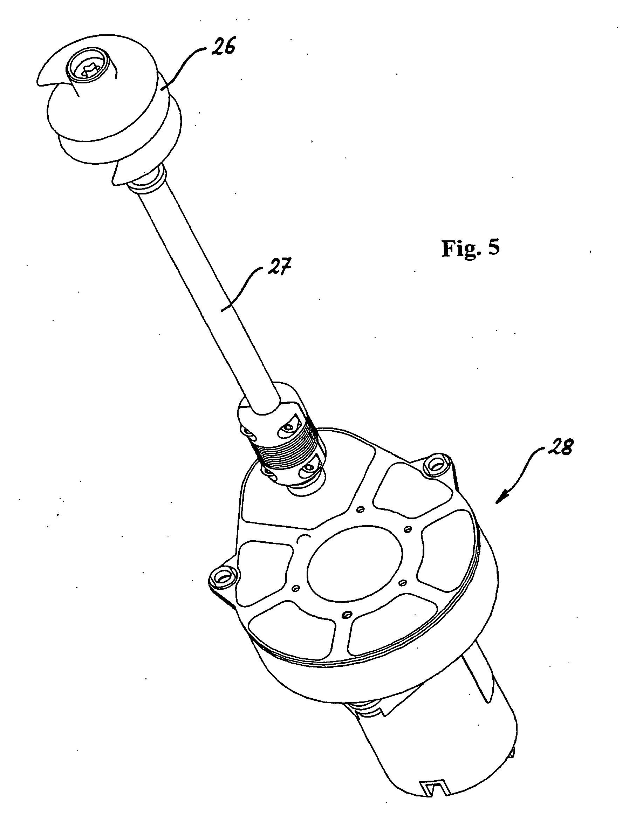

- **Fig. 5:**: Perspektivische Darstellung der Einfädeleinrichtung der Schlauchpumpe von Fig. 2.

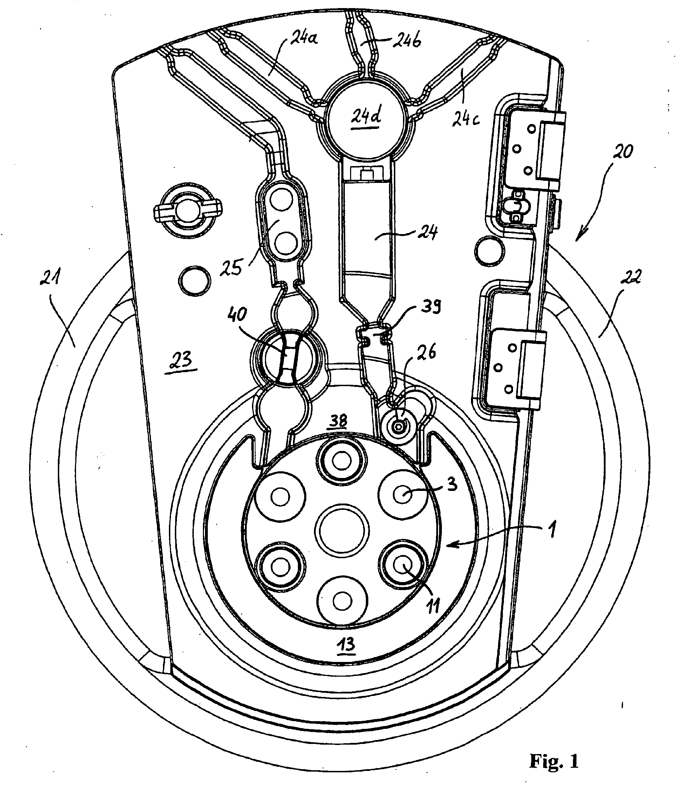

In Figur 1 ist der Injektionskopf einer Injektionsvorrichtung zur Injektion von zwei verschiedenen oder gleichen Kontrastmitteln und einer NaCl-Spüllösung in die Blutbahn eines Patienten gezeigt, in der eine erfindungsgemäße Schlauchpumpe 1 verwendet wird. Solche Injektionsvorrichtungen werden bspw. zur Injektion von Kontrastmitteln bei der Durchführung von bildgebenden Verfahren wie Computertomographie, Ultraschalluntersuchungen und Magnetresonanztomographie (MRT) verwendet. Die Injektionsvorrichtung umfasst den in Figur 1 gezeigten Injektionskopf 20, in dem die Schlauchpumpe 1 angeordnet ist. Der Injektionskopf 20 umfasst ein Kunststoffgehäuse mit zwei kreisringförmigen Handgriffen 21, 22. Zwischen den Handgriffen 21 und 22 ist ein Panel 23 angeordnet, welches mit einem hier zeichnerisch nicht dargestellten Deckel verschließbar ist. Das Panel 23 weist in seinem unteren Bereich eine Ausnehmung zur Aufnahme der Schlauchpumpe 1 auf. Darüber befinden sich kanalförmige Ausnehmungen 24, 25, in welche eine verzweigte Schlauchanordnung (welche hier zeichnerisch nicht dargestellt ist) eingefügt werden kann. Bei der Schlauchanordnung handelt es sich insbesondere um eine Schlauchanordnung, wie sie in der EP 2 011 541 A2 im Detail beschrieben ist. Diese Schlauchanordnung umfasst insgesamt drei Zufuhrschläuche, nämlich einen ersten Zufuhrschlauch für ein erstes Kontrastmittel, einen zweiten Zufuhrschlauch für ein zweites Kontrastmittel und einen dritten Zufuhrschlauch für eine Spüllösung (insbesondere NaCl). Die drei Zufuhrschläuche werden an hier ebenfalls zeichnerisch nicht dargestellte Vorratsflaschen für die Kontrastmittel und die Spüllösung angeschlossen und in die im oberen Bereich des Panels 23 angeordneten Verzweigungen 24a, 24b und 24c der Ausnehmung 24 eingefügt. Die drei von den Vorratsbehältern kommenden Zufuhrschläuche werden über ein Verzweigungsstück, welches in der kreisförmigen Ausnehmung 24d des Panels 23 eingefügt wird, in einem Schlauchstück zusammengeführt, welches zur Schlauchpumpe 1 geführt wird.

Zur Einführung des Schlauchs in die Schlauchpumpe 1 ist eine Einfädeleinrichtung vorgesehen. Die Funktionsweise und die Ausgestaltung dieser Einfädeleinrichtung wird im Folgenden noch erläutert. Der Schlauch wird schließlich durch die Schlauchpumpe 1 geführt und in die Ausnehmung 25 im oberen, linken Teil des Panels 23 eingelegt. Das Schlauchende wird an einen Patientenschlauch angeschlossen, über den die in dem Schlauch geführten Medien schließlich in die Blutbahn des Patienten injiziert werden können. Zur Fixierung des Schlauchs auf dem Panel 23 ist eine Fixiereinrichtung vorgesehen, welche eine Fixierung des Schlauchs an einer ersten, eingangsseitigen Stelle 39 und wenigstens einer zweiten, ausgangseitigen Stelle 40 der Schlauchpumpe ermöglicht. An den Fixierstellen 39 und 40 sind zweckmäßig Ultraschallsensoren zur Erfassung von Luftblasen in dem Schlauch angeordnet. Weitere Fixierstellen des Schlauchs auf dem Panel 23 sind möglich und bspw. in der EP 2011541 A1 beschrieben.

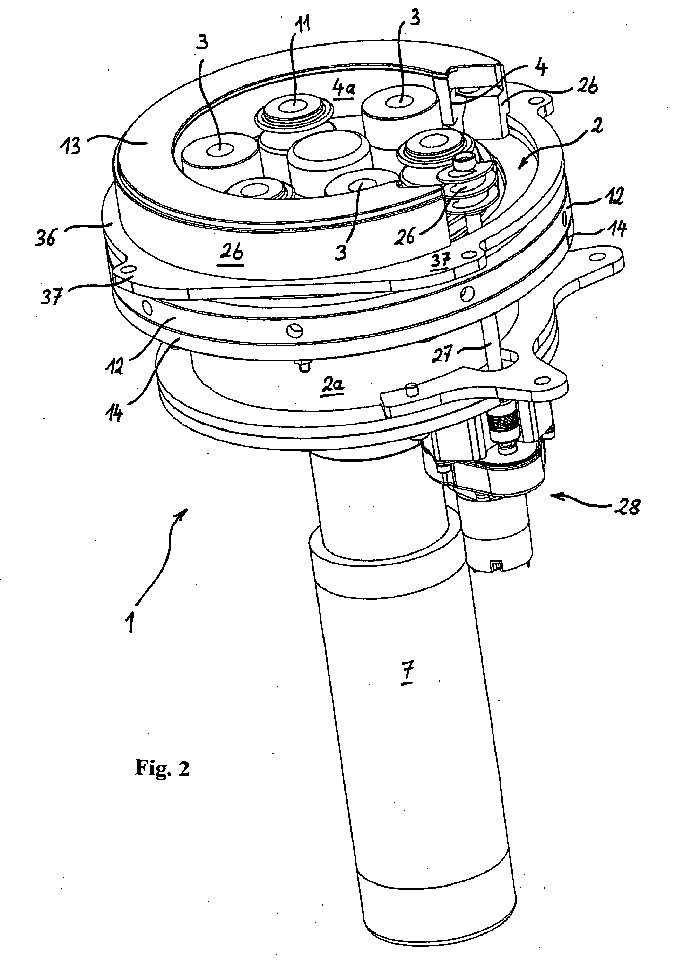

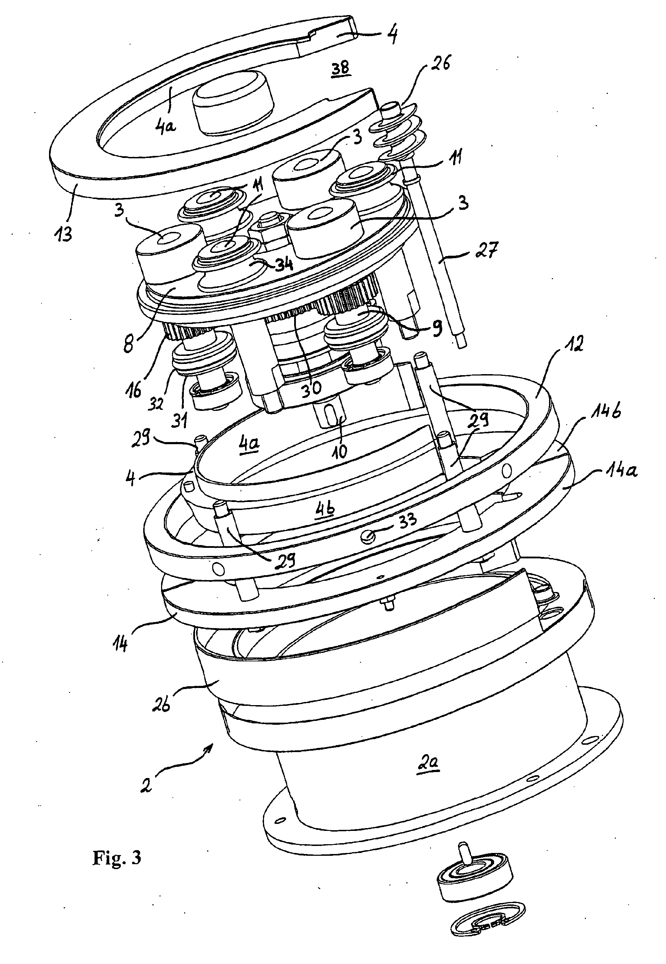

In den Figuren 2 und 3 ist die Schlauchpumpe 1 im Detail in einer perspektivischen Ansicht gezeigt, wobei Figur 3 eine Explosionsdarstellung ist. Die Schlauchpumpe 1 umfasst eine untere Pumpeneinheit mit einem Antriebsmotor 7, sowie eine obere Pumpeneinheit mit einem Gehäuse 2. Das Gehäuse 2 ist in einen unteren Gehäuseteil 2a und einen oberen Gehäuseteil 2b unterteilt. Der untere Gehäuseteil 2a kann mit dem oberen Gehäuseteil 2b einstückig oder auch zweiteilig ausgebildet sein.

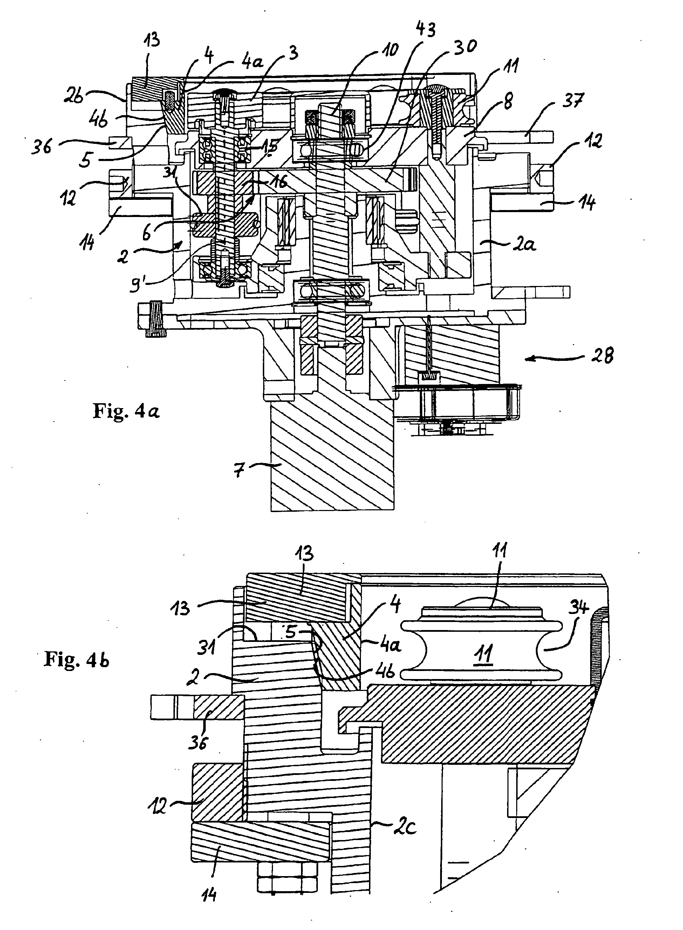

Die untere Pumpeneinheit umfasst den Antriebsmotor 7 mit einer Antriebswelle 10, welche über ein Getriebe mit der oberen Pumpeneinheit gekoppelt ist. Der Aufbau der oberen Pumpeneinheit ist aus der Schnittdarstellung der Figur 4 zu entnehmen. Im Innern des Gehäuses 2 ist ein an die Antriebswelle 10 des Antriebsmotors 7 gekoppeltes Getriebe 6 angeordnet. Das Getriebe umfasst ein Sonnenrad 30, welches drehfest mit der Antriebswelle 10 des Antriebsmotors 7 verbunden ist. Das obere Ende der Antriebswelle 10 ist über ein Lager 43 in einer Trägerscheibe 8 drehbar gelagert. Auf der Trägerscheibe 8 sind mehrere Quetschelemente 3 angeordnet. Bei den Quetschelementen 3 handelt es sich bei dem hier zeichnerisch dargestellten Ausführungsbeispiel um angetriebene Quetschrollen 3, wobei hier drei solcher Quetschrollen 3 gleichförmig am Außenumfang der kreisrunden Trägerscheibe 8 angeordnet sind. Die Quetschrollen 3 sind drehbar auf der Trägerscheibe 8 gelagert. Hierfür ist jede der drei Quetschrollen 3 auf einer Welle 9 mit einer Achse 9' aufgesetzt und jede Welle 9 ist über ein Lager 15 in einer Bohrung der Trägerscheibe 8 gelagert. Die Wellen 9 und damit die Achsen 9' der Quetschrollen 3 verlaufen parallel zur Antriebswelle 10 des Antriebsmotors 7. Der Antriebsmotor 7 versetzt die Trägerscheibe 8 und die Quetschrollen 3 bei laufender Pumpe über das Getriebe 6 in Drehung. Das Getriebe 6 umfasst neben dem Sonnenrad 30 Planetenräder 16, wobei jeder Quetschrolle 3 ein solches Planetenrad 16 zugeordnet und drehfest an der Welle 9 befestigt ist. Jedes der Planetenräder 16 ist über eine Zahnung an das Sonnenrad 30 des Planetengetriebes gekoppelt. An jeder Welle 9 ist neben dem Planetenrad 16 ein Reibrad 31 angeordnet, wobei das Reibrad 31 drehfest und im Abstand zum Planetenrad 16 an der Welle 9 befestigt ist. Am Außenumfang jedes Reibrads 31 ist eine umlaufende Nut 34 angeordnet, in welche ein Gummiring 32 (O-Ring) eingefügt ist. Über diesen Gummiring 32 steht das Reibrad 31 mit dem Innenumfang 2c des Pumpengehäuses 2 in Kontakt. Der Innenumfang 2c des Gehäuses 2 wirkt dadurch als Hohlrad eines Planetengetriebes. Wird die Antriebswelle 10 durch den Antriebsmotor 7 in Rotation versetzt, so wird diese Rotationsbewegung über die Kopplung des Planetenrads 16 am Sonnenrad 30 auf die Welle 9 übertragen, wodurch sich die Welle 9 und die drehfest mit dieser verbundene Quetschrolle 3 in Rotation versetzt. Gleichzeitig rollt das Reibrad 31 am Innenumfang 2c des Pumpengehäuses 2 ab, wodurch sich die Trägerscheibe 8 ebenfalls gegenüber dem Pumpengehäuse 2 in Drehung versetzt. Durch die Reibräder 31 kann die Trägerscheibe 8 von dem Antriebsmotor 7 auch dann in Rotation versetzt werden, wenn sich noch kein Schlauch in der Schlauchpumpe befindet.

Auf der Trägerscheibe 8 sind zusätzlich zu den Quetschrollen 3 noch Führungsrollen 11 gelagert. Die Führungsrollen 11 dienen zur Führung des Schlauchs zwischen benachbarten Quetschrollen 3 und sind nicht angetrieben. Am Außenumfang weisen die Führungsrollen 11 eine im Querschnitt halbkreisförmige Nut 34 auf, in welcher der Schlauch geführt wird. Die Anordnung der Führungsrollen 11 und der Quetschrollen 3 auf der Trägerscheibe 8 ist insbesondere der Explosionsdarstellung der Figur 3 zu entnehmen.

Zur Einführung des Schlauchs in die Schlauchpumpe ist eine Einfädeleinrichtung vorgesehen, welche den Schlauch selbsttätig zwischen den Quetschrollen 3 und dem Gegenlager 4 einfädelt. Die Einfädeleinrichtung umfasst eine außerhalb der Trägerscheibe 8 angeordnete Schneckenspindel 26. Die Schneckenspindel 26 ist auf einer Welle 27 angeordnet, wobei die Welle 27 parallel zur Achse 9' der Quetschrollen 3 verläuft. Die Welle 27 ist drehbar in einem Gehäuseteil 2 der Schlauchpumpe gelagert und an einen Spindelantrieb 28 gekoppelt, mit dem die Welle 27 und die Schneckenspindel 26 in Drehung versetzt werden kann, um einen in die Schneckenspindel eingelegten Schlauch in die Schlauchpumpe einzufädeln. Die oberen Schneckengänge der Schneckenspindel 26 ragen in Längsrichtung der Schlauchpumpe (also parallel zur Achse der Wellen 10 bzw. 27) über die Oberseite der Quetschrollen 3 und der Führungsrollen 11 hinaus.

Am oberen Ende der oberen Pumpeneinheit ist ein Gegenlager 4 angeordnet. Das Gegenlager 4 ist kreissegmentförmig mit einer Aussparung 38 ausgebildet und erstreckt sich zweckmäßig über einen Winkelbereich von 200° bis 300°. Die Schneckenspindel 26 ist im Bereich der Aussparung 38 des Gegenlagers 4 angeordnet. Das Gegenlager 4 verfügt über eine Wirkfläche 4a, welche dem Außenumfang der Quetschrollen 3 in einem Abstand d gegenüberliegt. In dem Spalt zwischen der Wirkfläche 4 und dem Außenumfang jeder Quetschrolle 3 wird der Schlauch eingefädelt.

Zum Einführen des Schlauchs in die Schlauchpumpe 1 wird das einzuführende Schlauchstück zunächst über die Fixiereinrichtung an den beiden Fixierstellen 39 und 40 auf dem Panel 23 fixiert. Das Schlauchstück zwischen den Fixiereinrichtungen 39 und 40 weist (aufgrund des Eigendralls des Schlauchstücks) dann die Form einer Schlaufe auf. Anschließend wird das Schlauchstück in die Schneckenspindel 26 eingelegt. Anschließend wird die Pumpe in Gang gesetzt, wodurch der Antriebsmotor 7 die Trägerscheibe 8 in Rotation versetzt. Gleichzeitig setzt der Spindelantrieb 28 die Schneckenspindel 26 in Drehung. Hierfür ist der Spindelantrieb 28 mit der Steuerung des Antriebsmotors 7 gekoppelt. Durch die Drehung der Schneckenspindel 26 wird der Schlauch von der Schneckenspindel 26 nach unten in Richtung der Trägerscheibe 8 geführt. Durch die Drehung der Trägerscheibe wird eine der Führungsrollen 11 auf den Schlauch zubewegt und der Schlauch greift in die Nut 34 am Außenumfang der Führungsrolle 11 ein. Durch die weitere Drehung der Trägerscheibe 8 bewegt sich die darauf angeordnete Führungsrolle 11 in Förderrichtung der Pumpe weiter und zieht dabei den Schlauch durch Kraftschluss in der Nut 34 zum einen nach unten in Richtung der Trägerscheibe 8 und drückt ihn zum anderen durch Formschluss in radialer Richtung nach außen gegen das Gegenlager 4. Bei weiterer Drehung der Trägerscheibe 8 zieht die Führungsrolle 11 den Schlauch aufgrund der Haftreibung an der Schlauchoberfläche und des Kraftschlusses in der Nut 34 an ihrem Außenumfang weiter in die Schlauchpumpe entlang des Innenumfangs des kreissegmentförmig ausgebildeten Gegenlagers 4 ein, bis die Trägerscheibe mit der darauf angeordneten Führungsrolle 11 (nahezu) eine volle Umdrehung ausgeführt hat und der Schlauch durch die weitere Drehung der Trägerscheibe vollständig in die Schlauchpumpe eingezogen worden ist. Durch die Drehung der Trägerscheibe wird der Schlauch schließlich von der auf der Trägerscheibe 8 der Führungsrolle 11 folgenden Quetschrolle 3 schließlich gegen das Gegenlager 4 gequetscht. Auf diese Weise wird der Schlauch selbsttätig zwischen dem Außenumfang der Quetschrollen 3 und dem Gegenlager 4 eingeführt und bei weiterer Drehung der Trägerscheibe 8 zur Förderung der darin geführten Flüssigkeit gequetscht.

Ist der Schlauch vollständig in der Schlauchpumpe eingeführt, drücken die Quetschrollen 3 den Schlauch bei laufender Schlauchpumpe (also bei rotierender Trägerscheibe 8 und rotierenden Quetschrollen 3) unter Quetschung des Schlauchdurchmessers gegen die Wirkfläche 4a des Gegenlagers 4, um dadurch das in dem Schlauch geführte Medium in Förderrichtung (d.h. in Drehrichtung der Trägerscheibe 8) weiteizufördern.

Nach Beendigung des Pumpvorgangs kann die Einfädeleinrichtung bei einem erforderlichen Schlauchwechsel auch zum Ausfädeln des verbrauchten Schlauchs verwendet werden. Hierzu wird der Spindelantrieb 28 bei laufender Schlauchpumpe in umgekehrter Drehrichtung betrieben. Dadurch zieht die Schneckenspindel 26 das in die Schlauchpumpe eingelegte Schlauchstück nach oben, so dass der Eingriff des Schlauchs in der Nut 34 der Führungsrollen 11 gelöst wird. Nach einer vollen Umdrehung der Trägerscheibe ist der Schlauch vollständig aus der Schlauchpumpe heraus gezogen und kann nach Lösen der Fixierungen an den Fixierstellen 39 und 40 entnommen und durch einen neuen Schlauch ersetzt werden. Zur Einleitung der Ausfädelung eines verbrauchten Schlauchs ist eine Steuerroutine in der. Steuerung des Spindelantriebs 28 vorgesehen, welche auf Knopfdruck vom Bediener ausgelöst werden kann.

Zur optimalen Einstellung des Abstands zwischen dem Gegenlager 4 und den Quetschrollen 3 ist das Gegenlager in einem bevorzugten Ausführungsbeispiel mit seiner Wirkfläche 4a gegenüber den Quetschrollen 3 verschiebbar am Gehäuse 2 angeordnet. Hierfür ist das Gegenlager 4 mit einem Druckring 13 verbunden. Bei dem Druckring 13 handelt es sich ebenfalls um einen kreissegmentförmigen Ring. Das Gegenelement 4 weist eine der Wirkfläche 4a gegenüberliegende Stellfläche 4b auf. Diese ist konisch bzw. kegelförmig ausgebildet. Die aus dem Gegenelement 4 und dem Druckring 13 bestehende Anordnung ist in der oberen Öffnung des Gehäuses 2 so angeordnet, dass sich die konische Stellfläche 4b des Gegenelements 4 gegen eine komplementär (also ebenfalls konisch bzw. kegelförmig) geformte Stützfläche 5 am Gehäuse 2 abstützt, wobei sich die Stützfläche 5 am Gehäuse 2 nach unten (also ins Gehäuseinnere) hin konisch erweitert (Figur 4, links oben).

Auf der Außenseite des Gehäuses 2 ist ein am Gehäuse befestigter Befestigungsring 36 (welche in Figur 3 aus Gründen der Übersichtlichkeit weg gelassen worden ist) mit Befestigungsflanschen 37 zur Befestigung des Gehäuses 2 am Panel 23 des Injektionskopfs 20 vorgesehen. Auf der Außenseite des Gehäuses 2 ist weiterhin im Übergangsbereich zwischen dem unteren Gehäuseteil 2a und dem oberen Gehäuseteil 2b ein Stellring 12 angeordnet. Bei dem Stellring 12 handelt es sich um einen Kreisring, der an seiner Kreisinnenfläche über ein Innengewinde verfügt. Auf der Außenseite des Gehäuses 2 ist ein zu diesem Innengewinde komplementäres Außengewinde vorgesehen. Der Stellring ist über diese Gewindeanordnung an das Gehäuse 2 derart gekoppelt, dass durch eine Verdrehung des Stellrings 12 gegenüber dem Gehäuse 2 der Stellring in axialer Richtung kontinuierlich zwischen einer oberen Grenzstellung und einer unteren Grenzstellung bezüglich des Gehäuses 2 verschiebbar ist. Zum Verdrehen des Stellrings 12 gegenüber dem Gehäuse 2 sind am Außenumfang des Stellrings 12 mehrere Bohrungen 33 vorgesehen, in die ein Stift eingreifen kann.

An der Unterseite des Stellrings 12 liegt ein Verschiebering 14 an. Der Verschiebering 14 ist aus zwei halbkreisförmigen Ringsegmenten 14a und 14b zusammengesetzt und über mehrere Bolzen 29 mit dem Druckring 13 verbunden (Figur 3).

Über die Anordnung, bestehend aus dem Gegenlager 4, dem Druckring 13, dem Verschiebering 14 und dem Stellring 12 kann der Abstand d zwischen den Quetschrollen 3 und der Wirkfläche 4a des Gegenlagers 4 eingestellt werden.

Um den Abstand d zwischen dem Außenumfang der Quetschrollen 3 und der Wirkfläche 4a zu maximieren, wird das Gegenlager 4 in seine erste (obere) Grenzstellung gebracht. Hiervon ausgehend, kann der Abstand d verkleinert werden, indem der Stellring 12 am Gehäuse 2 in Richtung seiner unteren Grenzstellung gedreht wird. Dadurch verschiebt sich der Stellring 12 von seiner oberen Grenzstellung nach unten. Dadurch wird auch der an der Unterseite des Stellrings 12 anliegende Verschiebering 14 gegenüber dem Gehäuse nach unten verschoben. Da der Verschiebering 14 über die Bolzen 29 mit dem Druckring 13 verbunden ist, verschiebt sich dadurch auch der Druckring 13 mit dem daran befestigten Gegenlager 4 nach unten. Dabei gleitet die Stellfläche 4b des Gegenlagers 4 auf der kegelförmigen Stützfläche 5 am Gehäuse 2 entlang. Bei dieser Bewegung zieht sich das kreissegmentförmige Gegenlager 4 leicht zusammen und verringert ihren Durchmesser, wodurch die Wirkfläche 4a in radialer Richtung auf die Quetschrollen 3 bzw. die Führungsrollen 11 zu gedrückt wird. Durch diese Bewegung verringert sich der Abstand d zwischen der Wirkfläche 4a und dem Außenumfang der Quetschrollen 3. Ist der Stellring 12 bei weiterer Drehung in seiner unteren Grenzstellung angelangt, sitzt die Unterseite des Druckrings 13 auf einem Sockel 31 des Gehäuses 2 auf. In dieser Stellung ist der Abstand d zwischen der Wirkfläche 4a und dem Außenumfang der Quetschrollen 3 bzw. der Führungsrollen 11 in Minimalstellung.

Durch diese Anordnung des Gegenlagers 4 kann das Spaltmaß (also der Abstand d) zwischen der Wirkfläche 4a und dem Außenumfang der Quetschrollen 3 auf einen für den Betrieb der Pumpe optimalen Wert eingestellt werden. Diese Einstellung erfolgt erstmalig vor der Inbetriebnahme der Schlauchpumpe. Zur Einstellung eines gewünschten Abstand d wird zweckmäßig eine Lehre verwendet, deren Dicke dem einzustellenden Spaltmaß entspricht und welche zwischen der Wirkfläche 4a und dem Außenumfang der Quetschrollen 3 eingelegt wird. Anschließend wird der Stellring 12 gegenüber dem Gehäuse verdreht, bis die. Wirkfläche 4a und der Außenumfang der Quetschrollen 3 an den Außenflächen der Lehre anliegen. Das Spaltmaß kann im Bedarfsfall bei einer Wartung der Schlauchpumpe nachgestellt werden.

Die Erfindung ist nicht auf das beschriebene Ausführungsbeispiel beschränkt. So kann die Erfindung nicht nur in Radial-Schlauchpumpen sondern bspw. auch in Schlauchpumpen mit einem linearen Wirksystem, wie z.B. in sog. linearen Peristaltikpumpen bzw. Wanderwellenpumpen, verwendet werden. Der Einsatz der erfindungsgemäßen Schlauchpumpe ist ferner nicht auf Injektionsvorrichtungen beschränkt, sondern erstreckt sich auch auf andere Pumpeinrichtungen, wie z.B. Infusionspumpen. Die Einfädeleinrichtung kann statt der Schneckenspindel auch einen Greifer mit einem linearen Antrieb aufweisen, wobei der Greifer den in die Einfädeleinrichtung eingelegten Schlauch bzw. das eingelegte Schlauchstück ergreift oder umgreift und der lineare Antrieb den Schlauch dann nach unten in Richtung der Trägerscheibe führt, so dass dieser von den Führungsrollen, wie oben beschrieben, mitgeführt und um das Gegenlager herum in die Schlauchpumpe zwischen den Quetschrollen und dem Gegenlager eingeführt werden kann. Der lineare Antrieb kann dabei über einen Linearmotor oder über einen Rotationsmotor mit Übersetzungsgetriebe zur Umsetzung der Drehbewegung in einen Linearantrieb bereit gestellt werden. Alternativ zu einem motorischen Antrieb kann auch ein bistabiler Magnet verwendet werden, mit dem die Einfädeleinrichtung in Bewegung gesetzt werden kann, um den Schlauch in die Pumpe einzuführen. Statt den Führungsrollen können auch Halteflügel zum Einsatz kommen, welche auf der Trägerscheibe angeordnet sind und das von der Einfädeleinrichtung in die Pumpe eingeführte Schlauchstück nach unten in Richtung der Trägerscheibe und radial nach außen in Richtung des Gegenlager drücken.

## Patentansprüche

1. Schlauchpumpe (1) zur Förderung eines in einem Schlauch geführten Mediums, mit mehreren Quetschelementen (3), welche den Schlauch unter Quetschung des Schlauchs gegen ein Gegenlager (4) drücken und dadurch das Medium in dem Schlauch in Förderrichtung weiter fördern, **dadurch gekennzeichnet, dass** die Schlauchpumpe über eine Einfädeleinrichtung zum automatischen Einrühren des Schlauchs zwischen den Quetschelementen (3) und dem Gegenlager (4) verfügt, wobei die Einfädeleinrichtung eine Schneckenspindel (26) umfasst.

2. Schlauchpumpe nach Anspruch 1, **dadurch gekennzeichnet, dass** die Quetschelemente durch Quetschrollen (3) gebildet sind, welche über ein Getriebe (6) von einem Antriebsmotor (7) in Rotation versetzt werden.

3. Schlauchpumpe nach Anspruch 2, **dadurch gekennzeichnet, dass** die Quetschrollen (3) auf einer Trägerscheibe (8) gelagert sind, wobei die Achse (9') jeder Quetschrolle (3) parallel zur Antriebswelle (10) des Antriebsmotor (7) verläuft.

4. Schlauchpumpe nach Anspruch 2 oder 3, **dadurch gekennzeichnet, dass** sowohl die Trägerscheibe (8) als auch die darin drehbar gelagerten Quetschrollen (3) bei laufender Schlauchpumpe über ein Getriebe (6) von dem Antriebsmotor (7) in Rotation versetzt werden.

5. Schlauchpumpe nach Anspruch 3 oder 4, **dadurch gekennzeichnet, dass** auf der Trägerscheibe (8) zwischen zwei benachbarten Quetschrollen (3) jeweils eine Führungsrolle (11) angeordnet ist.

6. Schlauchpumpe nach einem der Ansprüche 3 bis 5, **dadurch gekennzeichnet, dass** die Einfädeleinrichtung außerhalb der Trägerscheibe (8) angeordnet ist.

7. Schlauchpumpe nach einem der Ansprüche 1 bis 6, **dadurch gekennzeichnet, dass** die Schneckenspindel (26) von einem Spindelantrieb (28) drehend antreibbar ist.

8. Schlauchpumpe nach Anspruch 7, **dadurch gekennzeichnet, dass** der Spindelantrieb (28) so mit dem Antriebsmotor (7) gekoppelt ist, dass der Spindelantrieb (28) die Schneckenspindel (26) in Drehung versetzt, sobald der Antriebsmotor (7) die Trägerscheibe (8) in Rotation versetzt.

9. Schlauchpumpe nach einem der Ansprüche 1 bis 8, **dadurch gekennzeichnet, dass** die Schneckenspindel (26) drehfest auf einer Welle (27) angeordnet ist, wobei die Welle (27) parallel zur Achse (9') der Quetschrollen (3) verläuft.

10. Schlauchpumpe nach Anspruch 9, **dadurch gekennzeichnet, dass** die Welle (27) drehbar in einem Gehäuseteil (2) der Schlauchpumpe gelagert ist.

11. Schlauchpumpe nach einem der voranstehenden Ansprüche, **dadurch gekennzeichnet, dass** die Einfädeleinrichtung einen darin eingelegten Schlauch bei laufender Schlauchpumpe selbsttätig zwischen den Quetschelementen (3) und dem Gegenlager (4) einrührt.

12. Schlauchpumpe nach einem der voranstehenden Ansprüche, dadurch gekenntzeichnet, dass eine Fixiereinrichtung zur Fixierung des in die Schlauchpumpe einzuführenden Schlauchs vorgesehen ist, wobei die Fixiereinrichtung eine Fixierung des Schlauchs an einer ersten, eingangsseitigen Stelle (39) und einer zweiten, ausgangseitigen Stelle (40) der Schlauchpumpe ermöglicht.

13. Schlauchpumpe nach einem der voranstehenden Ansprüche, **dadurch gekennzeichnet, dass** das Gegenlager (4) als kreissegmentförmiger Ring mit einer Aussparung (38) ausgebildet ist und dass die Schneckenspindel (26) im Bereich der Aussparung (38) angeordnet ist.

14. Schlauchpumpe (1) nach einem der Ansprüche 3 bis 13, **dadurch gekennzeichnet, dass** das Getriebe (6) ein Sonnenrad (30) und wenigstens ein drehfest mit der jeweiligen Quetschrolle (3) verbundenes Planetenrad (16) umfasst und die Trägerscheibe (8) über ein Planetengetriebe, welches das Sonnenrad (30) und wenigstens ein Planetenrad (16, 31) für jede Quetschrolle (3) umfasst, von dem Antrieb (7) bei laufender Pumpe in Drehung versetzt wird, wobei das Planetenrad (16, 31) mit dem als Hohlrad wirkenden Innenumfang (2c) des Gehäuses (2) gekoppelt ist.

15. Schlauchpumpe nach Anspruch 14, dadurch gekenntzeichnet, dass das Sonnenrad (30) drehfest mit einer Antriebswelle (10) des Antriebs (7) verbunden ist und das von der Antriebswelle (10) auf das Sonnenrad (30) übertragene Drehmoment vom Sonnenrad (30) über den als Hohlrad wirkenden Innenumfang (2c) des fest stehenden Gehäuses (2) auf den Träger (8) übertragen wird, wodurch der Träger (8) bei laufender Pumpe auch ohne einen eingelegten Schlauch in Drehung versetzt wird.

## Claims

1. Tube pump (1) for conveying a medium carried in a tube, with a plurality of squeezing elements (3) which press the tube the tube against an abutment (4) and in so doing squeeze the tube and convey the medium in the tube further in the conveying direction, **characterised in that** the tube pump has a threading device for automatically introducing the tube between the squeezing elements (3) and the abutment (4), the threading device comprising a worm spindle (26).

2. Tube pump according to claim 1, **characterised in that** the squeezing elements are formed by squeezing rollers (3) which are set in rotation by a driving motor (7) through a gear unit (6).

3. Tube pump according to claim 2, **characterised in that** the squeezing rollers (3) are mounted on a carrier disk (8), the shaft (9) of each squeezing roller (3) running parallel to the driving shaft (10) of the driving motor (7).

4. Tube pump according to claim 2 or 3, **characterised in that** when the tube pump is running, both the carrier disk (8) and the squeezing rollers (3) mounted rotatably in it are set in rotation by the driving motor (7) through a gear unit (6).

5. Tube pump according to claim 3 or 4, **characterised in that** in each case a guiding roller (11) is arranged on the carrier disk (8) between two neighbouring squeezing rollers (3).

6. Tube pump according to one of the claims 3 to 5, **characterised in that** the threading device is arranged outside the carrier disk (8).

7. Tube pump according to one of claims 1 to 6, **characterised in that** the worm spindle (26) can be driven in rotation by a spindle drive (28).

8. Tube pump according to claim 7, **characterised in that** the spindle drive (28) is coupled with the driving motor (7) so that the spindle drive (28) sets the worm spindle (26) in rotation as soon as the driving motor (7) sets the carrier disk (8) in rotation.

9. Tube pump according to one of claims 1 to 8, **characterised in that** the worm spindle (26) is fixed so that it cannot turn on a shaft (27), the shaft (27) running parallel to the shaft (9') of the squeezing rollers (3).

10. Tube pump according to claim 9, **characterised in that** the shaft (27) is mounted rotatably in a part of the housing (2) of the tube pump.

11. Tube pump according to one of the preceding claims, **characterised in that** when the tube pump is running, the threading device automatically introduces a tube placed therein between the squeezing elements (3) and the abutment (4).

12. Tube pump according to one of the preceding claims, **characterised in that** a fixing device is provided for fixing the tube to be introduced into the tube pump, the fixing device allowing fixing of the tube at a first location (39) on the input side and a second location (40) on the output side of the tube pump.

13. Tube pump according to one of the preceding claims, **characterised in that** the abutment (4) is embodied as a ring in the shape of a circle segment with an opening (38) and **in that** the worm spindle (26) is arranged in the area of the opening (38).

14. Tube pump (1) according to one of claims 3 to 13, **characterised in that** the gear unit (6) comprises a sun wheel (30) and at least one planet gear (16) fixed to the respective squeezing roller (3) so that it cannot turn, and when the pump is running the carrier disk (8) is set in rotation by the drive (7) through a planetary gear train which comprises the sun wheel (30) and at least one planet gear (16, 31) for each squeezing roller (3), the planet gear (16, 31) being coupled with the internal circumference (2c) of the housing (2) which serves as annulus.

15. Tube pump according to claim 14, **characterised in that** the sun wheel (30) is fixed to a driving shaft (10) of the drive (7) so that it cannot turn and the torque transmitted by the driving shaft (10) to the sun wheel (30) is in turn transmitted by the sun wheel (30) through the internal circumference (2c) of the stationary housing (2) serving as annulus to the carrier (8), through which the carrier (8) is set in rotation even when no tube is placed therein when the pump is running.

## Revendications

1. Pompe péristaltique (1) servant à refouler un milieu guidé dans un tuyau flexible, comprenant plusieurs éléments d'écrasement (3), qui compriment le tuyau flexible tout en l'écrasant contre un contre-palier (4) et continuent à refouler ce faisant le milieu dans le tuyau flexible dans la direction de refoulement, **caractérisée en ce que** la pompe péristaltique dispose d'un dispositif de mise en place servant à introduire automatiquement le tuyau flexible entre les éléments d'écrasement (3) et le contre-palier (4), sachant que le dispositif de mise en place comprend une broche à vis sans fin (26).

2. Pompe péristaltique selon la revendication 1, **caractérisée en ce que** les éléments d'écrasement sont formés par des galets d'écrasement (3), qui sont amenés en rotation par un moteur d'entraînement (7), par l'intermédiaire d'un engrenage (6).

3. Pompe péristaltique selon la revendication 2, **caractérisée en ce que** les galets d'écrasement (3) sont logés sur un disque de support (8), tachant que l'axe (9') de chaque galet d'écrasement (3) s'étend de manière parallèle par rapport à l'arbre d'entraînement (10) du moteur d'entraînement (7).

4. Pompe péristaltique selon la revendication 2 ou 3, **caractérisée en ce qu'**aussi bien le disque de support (8) que les galets d'écrasement (3) logés de manière à pouvoir tourner dans ce dernier sont amenés en rotation par le moteur d'entraînement (7), par l'intermédiaire d'un engrenage (6) lorsque la pompe péristaltique fonctionne.

5. Pompe péristaltique selon la revendication 3 ou 4, **caractérisée en ce que** respectivement un galet de guidage (11) est disposé sur le disque de support (8) entre deux galets d'écrasement (3) adjacents.

6. Pompe péristaltique selon l'une quelconque des revendications 3 à 5, **caractérisée en ce que** le dispositif de mise en place est disposé à l'extérieur du disque de support (8).

7. Pompe péristaltique selon l'une quelconque des revendications 1 à 6, **caractérisée en ce que** la broche à vis sans fin (26) peut être entraînée en rotation par un mécanisme d'entraînement à broche (28).

8. Pompe péristaltique selon la revendication 7, **caractérisée en ce que** le mécanisme d'entraînement à broche (28) est couplé au moteur d'entraînement (7) de telle manière que le mécanisme d'entraînement à broche (28) amène en rotation la broche à vis sans fin (26) dès que le moteur d'entraînement (7) amène en rotation le disque de support (8).

9. Pompe péristaltique selon l'une quelconque des revendications 1 à 8, **caractérisée en ce que** la broche à vis sans fin (26) est disposée de manière solidaire en rotation sur un arbre (27), sachant que l'arbre (27) s'étend de manière parallèle par rapport à l'axe (9') des galets d'écrasement (3).

10. Pompe péristaltique selon la revendication 9, **caractérisée en ce que** l'arbre (27) est logé de manière à pouvoir tourner dans une partie de carter (2) de la pompe péristaltique.

11. Pompe péristaltique selon l'une quelconque des revendications précédentes, **caractérisée en ce que** le dispositif de mise en place introduit, lorsque la pompe péristaltique fonctionne, un tuyau flexible placé dans ledit dispositif de mise en place de manière autonome entre les éléments d'écrasement (3) et le contre-palier (4).

12. Pompe péristaltique selon l'une quelconque des revendications précédentes, **caractérisée en ce qu'**un dispositif de fixation est prévu aux fins de la fixation du tuyau flexible à introduire dans la pompe péristaltique, sachant que le dispositif de fixation permet une fixation du tuyau flexible au niveau d'un premier emplacement côté entrée (39) et d'un deuxième emplacement côté sortie (40) de la pompe péristaltique.

13. Pompe péristaltique selon l'une quelconque des revendications précédentes, **caractérisée en ce que** le contre-palier (4) est réalisé sous la forme d'une bague présentant une forme de segment de cercle dotée d'un évidement (38), et **en ce que** la broche à vis sans fin (26) est disposée dans la zone de l'évidement (38).

14. Pompe péristaltique (1) selon l'une quelconque des revendications 3 à 13, **caractérisée en ce que** l'engrenage (6) comprend un pignon solaire (30) et au moins un pignon satellite (16) relié de manière solidaire en rotation au galet d'écrasement (3) respectif, et **en ce que** le disque de support (8) est amené en rotation, lorsque la pompe fonctionne, par le mécanisme d'entraînement (7), par l'intermédiaire d'un engrenage planétaire, qui comprend le pignon solaire (30) et au moins un pignon satellite (16, 31) pour chaque galet d'écrasement (3), sachant que le pignon satellite (16, 31) est couplé à la périphérie intérieure (2c), ayant l'action d'une couronne, du carter (2).

15. Pompe péristaltique selon la revendication 14, **caractérisée en ce que** le pignon solaire (30) est relié de manière solidaire en rotation à un arbre d'entraînement (10) du mécanisme d'entraînement (7), et **en ce que** le couple de rotation transmis par l'arbre d'entraînement (10) sur le pignon solaire (30) est transmis sur le support (8) par le pignon solaire (30) par l'intermédiaire de la périphérie intérieure (2c), ayant l'action d'une couronne, du carter (2) immobile, ce qui permet d'amener en rotation le support (8), lorsque la pompe fonctionne, même en l'absence d'un tuyau flexible placé.
